# EUROPÄISCHE PATENTANMELDUNG

(11) **EP 1 532 939 A1**
(43) Veröffentlichungstag der Anmeldung: **25.05.2005**
(21) Anmeldenummer: 04027387.2
(22) Anmeldetag: 18.11.2004
(51) Int. Cl.: A61C 8/00, A61C 1/08, A61B 17/17

(54) **Verfahren und Vorrichtung zum Herstellen einer navigierten Bohrschablone für die Einbringung von Zahnimplantatbohrungen**

(30) Priorität: 18.11.2003 DE 10353913
(71) Anmelder: von Straelen, Frank, Dr., 46509 Xanten (DE)
(72) Erfinder: von Straelen, Frank, Dr., 46509 Xanten (DE)
(74) Vertreter: Viering, Jentschura & Partner

(57) **Zusammenfassung**

Die Erfindung betrifft ein Verfahren und eine Vorrichtung zum Herstellen einer Bohrschablone für die Einbringung von Zahnimplantatbohrungen in einen Patientenkiefer, wobei das Verfahren folgende Schritte aufweist: Bereitstellen eines dem Patientenkiefer anatomisch größengleich nachgebildeten Kiefermodells und eines ersten digitalen Datensatzes, welcher die anatomische Struktur des Patientenkiefers sowie für jede Zahnimplantatbohrung eine Sollposition und eine Sollorientierung definiert; Fixieren eines mit ersten Reflexionskörpern bestückten Referenzkörpers an dem Kiefermodell; Bohren von wenigstens einer Bohrung in das Kiefermodell unter Verwenden einer Bohreinrichtung, welche mit einer Mehrzahl von zweiten Reflexionskörpern bestückt ist, wobei von den ersten und zweiten Reflexionskörpern reflektierte Signale sensorgestützt zum Definieren der Relativposition und Relativorientierung von Bohreinrichtung und Kiefermodell in einem zweiten Datensatz erfasst werden und wobei die Führung der Bohreinrichtung auf Basis eines Vergleichs zwischen dem ersten und dem zweiten Datensatz erfolgt; Einbringen wenigstens einer Bohrhülse in jede Bohrung; und partielles Umgeben des die Bohrhülse tragenden Kiefermodells mit einer aushärtbaren Formgießmasse zum Ausbilden der Bohrschablone.

## Beschreibung

Die Erfindung betrifft ein Verfahren und eine Vorrichtung zum Herstellen einer navigierten Bohrschablone für die Einbringung von Zahnimplantatbohrungen.

Zahnimplantate werden zur Verankerung von Zahnprothesen oder von Einzelzähnen (in Form von künstlichen Zahnwurzeln) im Unter- und/oder Oberkiefer eines Patienten verwendet. Hierzu werden die in der Regel zylinderförmigen oder konischen Implantate in entsprechende Zahnimplantatbohrungen komplementärer Geometrie fest eingesetzt, im allgemeinen verschraubt. Die Zahnimplantatbohrungen müssen zuvor unter Betäubung in den betreffenden Patientenkiefer eingebohrt und gegebenenfalls für einen Gewindesitz des Zahnimplantates mit einem zum Zahnimplantat korrespondierenden Innengewinde versehen werden. Die korrekte Anordnung der Zahnimplantatbohrung innerhalb des Patientenkiefers ist von größter Bedeutung und verlangt insbesondere eine exakte Kenntnis der Kieferstruktur einschließlich des Nervengewebes sowie die Beachtung der erforderlichen Position und Abmessung des von dem Patienten benötigten Zahnersatzes. Schwierigkeiten bei der Einbringung solcher Zahnimplantatbohrungen resultieren insbesondere aus dem begrenzten, zur Platzierung der Bohrungen zur Verfügung stehenden Knochenangebotes sowie dem eingeschränkten Arbeitsraum für den chirurgischen Eingriff, so dass die Einbringung der Zahnimplantatbohrungen i.d.R. mittels aufwendiger technische Hilfsmittel erfolgt.

Zur Einbringung der Zahnimplantatbohrungen in den Kiefer des Patienten ist es bekannt, Navigationssysteme zu verwenden, mittels derer die Bohreinrichtung, basierend auf einer präoperativ aufgenommenen und durch geeignete Zielvorgaben für die Zahnimplantatpositionierung ergänzten röntgenographischen Darstellung des Patientenkiefers, navigationsgeführt durchgeführt wird, was manuell oder roboterunterstützt erfolgen kann. Solche navigiert am Patientenkiefer durchgeführte Bohrungen zeichnen sich zwar durch hohe Präzision aus, ihre Einbringung ist jedoch recht zeitaufwendig, wodurch die psychische und physische Belastung des Patienten vergrößert wird.

Des weiteren ist die Verwendung von Bohrschablonen bekannt, welche präoperativ angefertigt werden und eine zur Oberfläche des zu behandelnden Patientenkiefers im wesentlichen komplementäre Geometrie aufweisen. Zur Anfertigung der Bohrschablone wird eine präparierte Negativform des Patientenkiefers an den für die Implantatplatzierung vorgesehenen Positionen mit Markierungen versehen, oberhalb welcher als Bohrerführung dienende Hülsen eingebracht werden. Während des anschließenden chirurgischen Eingriffs werden die Zahnimplantatbohrungen dann mittels Führung des sterilen chirurgischen Bohrers durch die Bohrhülsen und direkt in den Kiefer eingebracht. Wenngleich das Arbeiten mit solchen Bohrschablonen gegenüber einer navigationsgeführten Bohrung am Patientenkiefer zeitsparend ist, lässt hierbei die Präzision der Platzierung und Orientierung der Zahnimplantatbohrungen häufig zu wünschen übrig, wobei diese intraoperativ in punkto Positionierung nicht mehr überprüfbar sind.

Eine Aufgabe der vorliegenden Erfindung ist es daher, ein Verfahren sowie eine Vorrichtung zum Herstellen einer Bohrschablone für die Einbringung von Zahnimplantatbohrungen in einen Patientenkiefer bereitzustellen, welche ein präzises und schnelles Arbeiten bei der Einbringung von Zahnimplantatbohrungen ermöglichen.

Diese Aufgabe wird gemäß den Merkmalen der unabhängigen Ansprüche gelöst.

Ein erfindungsgemäßes Verfahren zum Herstellen einer Bohrschablone für die Einbringung von Zahnimplantatbohrungen in einen Patientenkiefer weist folgende Schritte auf: Bereitstellen eines dem Patientenkiefer anatomisch größengleich nachgebildeten Kiefermodells und eines ersten digitalen Datensatzes, welcher die anatomische Struktur des Patientenkiefers sowie für jede Zahnimplantatbohrung eine Sollposition und eine Sollorientierung definiert; Fixieren eines mit ersten Reflexionskörpern bestückten Referenzkörpers an dem Kiefermodell; Bohren von wenigstens einer Bohrung in das Kiefermodell unter Verwenden einer Bohreinrichtung, welche mit einer Mehrzahl von zweiten Reflexionskörpern bestückt ist, wobei von den ersten und zweiten Reflexionskörpern reflektierte Signale sensorgestützt zum Definieren der Relativposition und Relativorientierung von Bohreinrichtung und Kiefermodell in einem zweiten Datensatz erfasst werden und wobei die Führung der Bohreinrichtung auf Basis eines Vergleichs des ersten und des zweiten Datensatzes erfolgt; Einbringen wenigstens einer Bohrhülse in jede Bohrung; und partielles Umgeben des die Bohrhülse tragenden Kiefermodells mit einer aushärtbaren Formgießmasse zum Ausbilden der Bohrschablone.

Infolge der Führung der Bohreinrichtung auf Basis eines Vergleichs zwischen dem zweiten, die Relativposition und - Orientierung von Bohreinrichtung und Kiefermodell darstellenden Datensatz und dem ersten, die Struktur des Patientenkiefers sowie die Soll-Position und/-Orientierung der Zahnimplantatbohrung definierenden Datensatz wird die betreffende Bohrung in dem Kiefermodell navigationsgestützt eingebracht, so dass die anschließend mit Hilfe des Kiefermodells gefertigte Bohrschablone bezüglich der Lage der Zahnimplantatbohrung eine hohe Präzision aufweist. Aufgrund der navigationsgestützten Herstellung der Bohrschablone kann während der anschließenden Einbringung der Zahnimplantatbohrung in den Patientenkiefer auf eine Navigationsführung verzichtet werden, ohne dass hierdurch die Präzision der Bohrung beeinträchtigt wird, wodurch die Dauer des erforderlichen chirurgischen Eingriffs erheblich verkürzt und die entsprechende Belastung des Patienten verringert wird. Wenngleich nicht erforderlich, kann eine Navigationsführung während des Eingriffs zur Positionskontrolle jedoch auch zusätzlich erfolgen, um etwaige doch vorhandene Abweichungen der Bohrungen in der Schablone von der Zielposition sofort korrigieren zu können (dies etwaigen Abweichungen liegen nicht in der navigierten Bohrung, sondern können Ihre Abweichung nur durch nicht exakte Positionierung der Schablone haben. Dies ist bei konventionellen Bohrschablonen wie oben ausgeführt nicht zu kontrollieren bzw. dann auch nicht zu korrigieren).

Das erfindungsgemäß zur Anfertigung der Schablone verwendete Kiefermodell stellt auch die für die Anfertigung des Zahnersatzes erforderliche Information über die spätere Implantatlage im Kieferknochen des Patienten bereit.
Die bei der erfindungsgemäßen Durchführung der navigationsgestützten Bohrung in ein dem Patientenkiefer anatomisch größengleich nachgebildetes Kiefermodell hat somit den weiteren Vorteil, dass das Kiefermodell und Einsetzen der betreffenden Implantate in die im Kiefermodell gefertigten Bohrungen bereits präoperativ zur Anfertigung der später dem Patienten einzusetzenden Provisorien(beispielweise provisorische Kronen und Brücken) verwendet werden kann. Dies hat den Vorteil, dass die bereits fertig hergestellten Provisorien dem Patienten unmittelbar nach der Operation eingesetzt werden können, sofern die Stabilität des Implantatsitzes dies zulässt.

Ein weiterer Vorteil des erfindungsgemäßen Verfahrens besteht in der dezentralen Anfertigung der Bohrschablone, die allein unter Zuhilfenahme des Kiefermodells sowie des den Patientenkiefer darstellenden Datensatzes im Labor angefertigt werden kann. Insbesondere ist es nicht erforderlich, dass der den Patienten behandelnde Implantologe, welcher schließlich die Einbringung der Zahnimplantatbohrung durchführt bzw. zuvor die Daten zur Darstellung der anatomischen Struktur des Patientenkiefers aufnimmt, selbst über die erfindungsgemäß verwendete Ausrüstung zur navigationsgeführten Anfertigung der Bohrschablone verfügt. Er stellt lediglich die aufgenommenen DVT-Daten sowie das Kiefermodell bereit und erhält zum gegebenen Zeitpunkt dann die im Labor navigationsgestützt angefertigte Bohrschablone zurück. Selbst die Anfertigung der DVT Aufnahme und die Anfertigung des Modells kann von dem Implantologen delegiert werden. Ein spezialisiertes Röntgenzentrum bzw. Dentallabor kann diese Schritte durchführen.

Gemäß einer bevorzugten Ausführungsform erfolgt das Bereitstellen des ersten digitalen Datensatzes auf Basis von DVT-Daten des Patientenkiefers.

Gemäß einer weiteren bevorzugten Ausführungsform werden als von den ersten und zweiten Reflexionskörpern reflektierte Signale elektromagnetische Signale verwendet, die von einer Sende-/Empfangseinrichtung emittiert und nach Reflexion an den ersten und zweiten Reflexionskörpern erfasst werden. Als elektromagnetische Signale werden bevorzugt optische Signale verwendet.

Gemäß einer weiteren bevorzugten Ausführungsform sind der Referenzkörper und die Bohreinrichtung jeweils mit drei ersten bzw. zweiten Referenzkörpern bestückt, welche jeweils in einer im wesentlichen T-förmigen Anordnung angeordnet sind.

Gemäß einer weiteren bevorzugten Ausführungsform wird als Sende-/Empfangseinrichtung eine CCD-Kamera verwendet.

Gemäß einer weiteren bevorzugten Ausführungsform weist der Referenzkörper einen am Kiefermodell in einer definierten Position fixierbaren Halteabschnitt und einen die ersten Reflexionskörper tragenden Reflexionsabschnitt auf.

Gemäß einer weiteren bevorzugten Ausführungsform weist der Reflexionsabschnitt eine im wesentlichen T-förmige Geometrie auf, wobei die ersten Reflexionskörper vorzugsweise an den Endpunkten des im wesentlichen T-förmigen Reflexionsabschnitts angeordnet sind.

Gemäß einer weiteren bevorzugten Ausführungsform beinhaltet der erste Datensatz ferner eine definierte Position des Referenzkörpers in dem Patientenkiefer.

Das Einbringen wenigstens einer Bohrhülse in jede Bohrung umfasst vorzugsweise folgende Schritte:
- formschlüssiges Einsetzen eines Stiftes in die Bohrung; und
- formschlüssiges Anordnen einer Bohrhülse über den Stift.

Gemäß einer bevorzugten Ausführungsform wird über der Bohrhülse eine weitere Bohrhülse formschlüssig angeordnet.

Vorzugsweise wird als aushärtbare Formgießmasse ein Polymer verwendet.

Vorzugsweise wird auf Basis des mit den Bohrungen versehenen Patientenkiefermodells ein Zahnersatz angefertigt.

Bei dem Verfahren zum Herstellen einer navigierten Bohrschablone wird vorzugsweise eine Bohreinrichtung verwendet, welche aufweist:
- eine Trageinheit, welche einen um einen ersten Aufhängungspunkt frei schwenkbaren Kiefermodellträger aufweist; und
- eine Bohreinheit, welche einen um einen zweiten Aufhängungspunkt frei schwenkbaren Bohrer zum Bohren in ein auf dem Kiefermodellträger angeordnetes Kiefermodell aufweist.

An dem Bohrer wird vorzugsweise ein mit der Mehrzahl der zweiten Reflexionskörper versehenes Trägerelement fixiert.

Das Trägerelement weist vorzugsweise eine im wesentlichen T-förmige Geometrie auf, wobei die zweiten Reflexionskörper bevorzugt an den Endpunkten des im wesentlichen T-förmigen Trägerelements angeordnet sind.

Gemäß einem weiteren Aspekt betrifft die Erfindung eine Bohrschablone für die Einbringung von Zahnimplantatbohrungen in einen Patientenkiefer, welche gemäß dem obigen Verfahren hergestellt ist, sowie auch einen Zahnersatz, welcher an einem mittels des obigen Verfahrens mit Bohrungen versehenen Patientenkiefermodells hergestellt worden ist.

Gemäß einem weiteren Aspekt betrifft die Erfindung eine Bohreinrichtung zum Bohren von wenigstens einer Bohrung in ein Kiefermodell, wobei die Bohreinrichtung aufweist:
- eine Trageinheit, welche einen um einen ersten Aufhängungspunkt frei schwenkbaren Kiefermodellträger aufweist; und
- eine Bohreinheit, welche einen um einen zweiten Aufhängungspunkt frei schwenkbaren Bohrer zum Bohren in ein auf dem Kiefermodellträger angeordnetes Kiefermodell aufweist.

Weitere Ausgestaltungen der Erfindung sind der nachfolgenden Beschreibung und den Unteransprüchen zu entnehmen.

Die Erfindung wird nachstehend anhand von in den beigefügten Abbildungen dargestellten Ausführungsbeispielen näher erläutert.

Es zeigen:
- Fig. 1: ein Flussdiagramm zur Erläuterung des erfindungsgemäßen Verfahrens zur Herstellung einer Bohrschablone; und
- Fig. 2: eine schematische Darstellung einer bevorzugten Ausführungsform einer bei der Durchführung des erfindungsgemäßen Verfahrens verwendeten Bohreinrichtung.

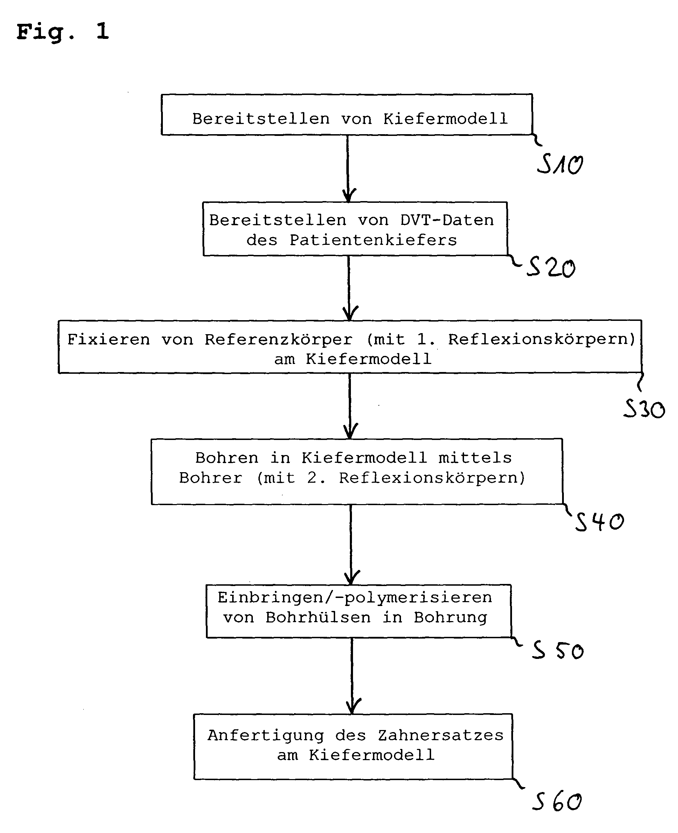

Gemäß Fig. 1 wird in einem ersten Schritt des erfindungsgemäßen Verfahrens ein dem Patientenkiefer anatomisch größengleich nachgebildetes Kiefermodell bereitgestellt (Schritt S10), welches vorzugsweise aus einem geeigneten Kunststoff sowie in grundsätzlich bekannter Weise erfolgt.

Außerdem wird ein erster digitaler Datensatz, welcher die anatomische Struktur des Patientenkiefers sowie für jede Zahnimplantatbohrung eine Sollposition und eine Sollorientierung definiert, bereitgestellt (Schritt S20) sowie auf einem digitalen Datenträger (z.B. CD-ROM) oder direkt auf der Festplatte eines PC's gespeichert. Dieser erste digitale Datensatz enthält vorzugsweise einen Satz DVT-Daten, welche mittels eines digitalen Volumen-Tomographen (DVT) aufgenommen werden und ähnlich wie Computertomograph-Daten (CT-Daten) ein dreidimensionales Abbild des Patientenkiefers bereitstellen.

Alternativ, jedoch im Hinblick auf die größere Bestrahlungsdauer und - Intensität weniger bevorzugt, können auch Daten einer herkömmlichen CT-Aufnahme zur schichtweisen Abbildung des Patientenkiefers aufgenommen und zur Erstellung eines dreidimensionalen Abbilds des Patientenkiefers verwendet werden. Vorzugsweise wird bereits während der Aufnahme der DVT- bzw. CT-Daten von Patienten ein nachfolgend noch detailliert erläuterter Referenzkörper am Patientenkiefer in einer definierten Position fixiert, welcher beim später erfolgenden Bohren in das Kiefermodell am Kiefermodell angebracht wird. Um eine definierte und exakt reproduzierbare Position sowohl in dem Patientenkiefer als auch an dem Patientenkiefermodell zu gewährleisten, weist dieser Referenzkörper wenigstens zwei röntgensichtbare (röntgenopake) Referenzpunkte auf. Infolgedessen enthält der digitale Datensatz über die Positionsangabe der Referenzpunkte auch die erforderliche Information über die definierte Position des Referenzkörpers im Patientenkiefer. Vorzugsweise nimmt der Referenzkörper bereits während der Aufnahme des ersten digitalen Datensatzes röntgensichtbare (röntgenopake) Zähne auf, welche bei der späteren Betrachtung der DVT-Daten auf dem Monitor erkennbar sind und zur Planung bzw. Raumausrichtung von Implantatachsen durch die Zahnkronen bzw. Occlusalflächen der Zähne verwendet werden können.

Anschließend wird der mit ersten Reflexionskörpern bestückte Referenzkörper an dem Kiefermodell fixiert (Schritt S30). Die Bestückung mit den Reflexionskörpern kann wahlweise vor oder auch nach der Aufnahme des ersten digitalen Datensatzes erfolgen. Der Referenzkörper weist einen am Kiefermodell in einer definierten Position fixierbaren Halteabschnitt und einen die ersten Reflexionskörper tragenden Reflexionsabschnitt von im wesentlichen T-förmiger Geometrie auf, an dessen Endpunkten die ersten Reflexionskörper angeordnet sind.

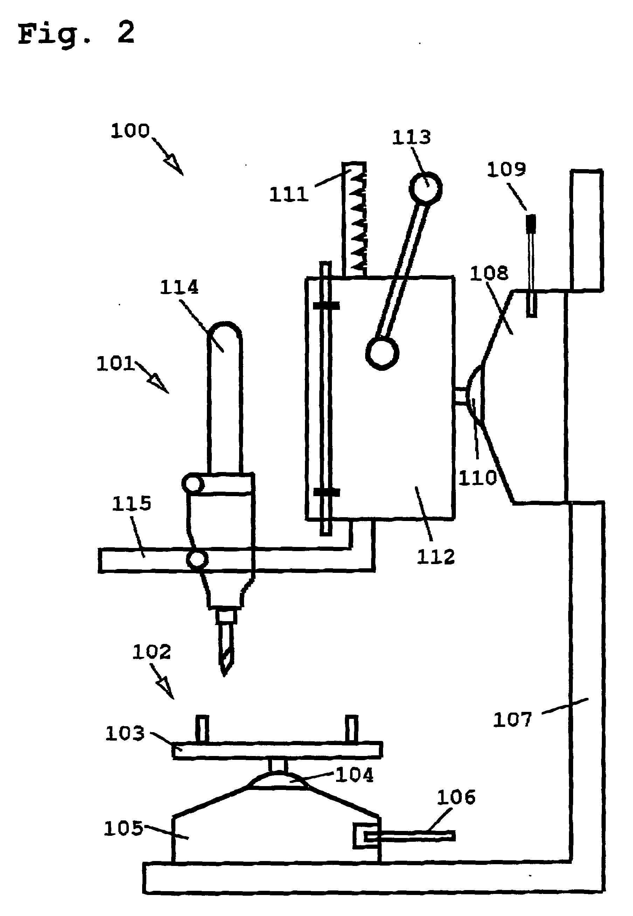

In das Kiefermodell wird wenigstens eine Bohrung gebohrt (Schritt S40), wobei hierzu vorzugsweise eine Bohreinrichtung verwendet wird, welche im Zusammenhang mit Fig. 2 noch genauer erläutert wird und zusätzlich mit einer Mehrzahl von zweiten Reflexionskörpern bestückt ist.

Eine CCD-Kamera wird verwendet, um Lichtimpulse zu emittieren und nach Reflexion an den ersten und zweiten Reflexionskörpern zu empfangen. Die von den ersten und zweiten Reflexionskörpern reflektierten Signale werden sensorgestützt in einem zweiten Datensatz erfasst, im welchem die Relativposition und Relativorientierung von Bohreinrichtung und Kiefermodell definiert wird.
Die Führung der Bohreinrichtung beim Bohren in das Kiefermodell erfolgt auf Basis eines Vergleichs zwischen dem ersten und dem zweiten Datensatz.

Hierzu kann während des Bohrens in das Kiefermodell der erste digitale Datensatz auf einem Monitor dargestellt werden, wobei dieser Darstellung aus dem während des Bohrvorganges ermittelten zweiten Datensatz die zur navigierten Führung der Bohreinrichtung benötigte Information über die Relativposition zwischen Bohreinrichtung und Kiefermodell in der Darstellung auf dem Monitor überlagert wird.

Dabei wird während des Bohrvorganges die CCD-Kamera in einer solchen Position schräg oberhalb der Bohreinrichtung angeordnet, dass sie sowohl mit den ersten Reflexionskörpern an dem Referenzkörper am Patientenkiefermodell als auch mit den zweiten Reflexionskörpern an der Bohreinrichtung Signale austauschen kann, d.h. dass die von der CCD-Kamera ausgesandten Signale von den ersten bzw. zweiten Reflexionskörpern reflektiert und dann wieder von der CCD-Kamera empfangen werden.

Nach wie oben navigationsgestützter Einbringung der Bohrungen in das Patientenkiefermodell wird in jede der Bohrungen ein passgenauer, d.h. randständiger Stift eingesetzt, über dem wiederum eine ebenfalls passgenaue Bohrhülse positioniert wird. Vorzugsweise wird über dieser Bohrhülse anschließend eine weitere Bohrhülse ebenfalls formschlüssig angeordnet. Daraufhin wird die gesamte Bohrhülsenanordnung durch Übergießen mit einem aushärtbaren Polymer zur Fertigstellung der Bohrschablone einpolymerisiert (Schritt S50).

Die Bohrhülsen können beispielsweise einen Innendurchmesser von 2 mm und 3 mm analog zu den entsprechenden Implantatbohrern aufweisen.

Vorzugsweise werden daraufhin an dem bereits mit den entsprechenden Bohrungen an den Zielpositionen versehenen Patientenkiefermodell Implantate inseriert und nach Einbringen der entsprechenden Aufbauten der geeignete, provisorische, festsitzende Zahnersatz oder definitive Prothesen angefertigt, so dass der Zahnersatz dem Patienten nach dem chirurgisch-operativen Eingriff unmittelbar eingesetzt werden kann, sofern die Primärstabilität der Implantate dies zulässt.

Bei zahnlosen Kiefern und teilbezahnten Kiefern, auf denen eine Positionierung der Schablone nicht möglich ist, besteht auch die Möglichkeit der Fixierung durch Kieferbruchosteosyntheseschrauben. Diese werden lateral durch die Schablone in den Kiefer geschraubt.

Gemäß Fig. 2 ist eine bevorzugte Ausführungsform einer zur Durchführung der Bohrungen in das Patientenkiefermodell verwendeten Bohreinrichtung dargestellt. Die Bohreinrichtung 100 weist ein Bohreinheit 101 und eine Trägereinheit 102 auf. Die Trägereinheit 102 weist einen Kiefermodellträger 103 auf, welcher über ein Kugelgelenk 104 auf einem Sockel 105 frei im Raum schwenkbar gelagert ist. An dem Sockel 105 ist ferner ein Feststellhebel 106 zum Fixieren des Kugelgelenks 104 bzw. des Kiefermodellträgers 103 in einer erwünschten Position angeordnet.

Ein den Sockel 105 tragendes Stativ 107 trägt ferner ein Halteelement 108 für ein ebenfalls über einen Feststellhebel 109 in einer gewünschten Position fixierbares weiteres Kugelgelenk 110, über welches ein entlang einer Zahnstange 111 höhenverstellbarer Träger 112 frei im Raum schwenkbar angelenkt ist, wobei zur Auf- bzw. Abwärtsbewegung des Trägers 112 ein Hebel 113 vorgesehen ist. Der Träger 112 trägt einen dem Kiefermodellträger 103 zugewandten Bohrer 114 in einer dafür vorgesehenen Halterung 115. Die Halterung 115 ist so ausgebildet, dass sie gleichzeitig zur Aufnahme des (nicht dargestellten) Referenzkörpers dient.

## Patentansprüche

1. Verfahren zum Herstellen einer navigierten Bohrschablone für die Einbringung von Zahnimplantatbohrungen in einen Patientenkiefer, wobei das Verfahren folgende Schritte aufweist:
• Bereitstellen eines dem Patientenkiefer anatomisch größengleich nachgebildeten Kiefermodells und eines ersten digitalen Datensatzes, welcher die anatomische Struktur des Patientenkiefers sowie für jede Zahnimplantatbohrung eine Sollposition und eine Sollorientierung definiert;
• Fixieren eines mit ersten Reflexionskörpern bestückten Referenzkörpers an dem Kiefermodell;
• Bohren von wenigstens einer Bohrung in das Kiefermodell unter Verwenden einer Bohreinrichtung, welche mit einer Mehrzahl von zweiten Reflexionskörpern bestückt wird, wobei von den ersten und zweiten Reflexionskörpern reflektierte Signale sensorgestützt zum Definieren der Relativposition und Relativorientierung von Bohreinrichtung und Kiefermodell in einem zweiten Datensatz erfasst werden und wobei die Führung der Bohreinrichtung auf Basis eines Vergleichs zwischen dem ersten und dem zweiten Datensatz erfolgt;
• Einbringen wenigstens einer Bohrhülse in jede Bohrung; und
• partielles Umgeben des die Bohrhülse tragenden Kiefermodells mit einer aushärtbaren Formgießmasse zum Ausbilden der Bohrschablone.

2. Verfahren nach Anspruch 1, wobei das Bereitstellen des ersten digitalen Datensatzes auf Basis von DVT-Daten des Patientenkiefers erfolgt.

3. Verfahren nach Anspruch 1 oder 2, wobei als von den ersten und zweiten Reflexionskörpern reflektierte elektromagnetische Signale verwendet werden, die von einer Sende-/Empfangseinrichtung emittiert und nach Reflexion an den ersten und zweiten Reflexionskörpern erfasst werden.

4. Verfahren nach Anspruch 3, wobei als elektromagnetische Signale optische Signale verwendet werden.

5. Verfahren nach einem der vorhergehenden Ansprüche, wobei der Referenzkörper und die Bohreinrichtung jeweils mit drei ersten bzw. zweiten Referenzkörpern bestückt sind.

6. Verfahren nach Anspruch 4 oder 5, wobei als Sende-/Empfangseinrichtung eine CCD-Kamera verwendet wird.

7. Verfahren nach einem der vorhergehenden Ansprüche, wobei der Referenzkörper einen am Kiefermodell in einer definierten Position fixierbaren Halteabschnitt und einen die ersten Reflexionskörper tragenden Reflexionsabschnitt aufweist.

8. Verfahren nach Anspruch 7, wobei der Reflexionsabschnitt eine im wesentlichen T-förmige Geometrie aufweist.

9. Verfahren nach Anspruch 8, wobei die ersten Reflexionskörper an den Endpunkten des im wesentlichen T-förmigen Reflexionsabschnitts angeordnet sind.

10. Verfahren nach einem der vorhergehenden Ansprüche, wobei der erste Datensatz ferner Daten aufweist, welche eine definierte Position des Referenzkörpers in dem Patientenkiefer beschreiben.

11. Verfahren nach einem der vorhergehenden Ansprüche, wobei das Einbringen wenigstens einer Bohrhülse in jede Bohrung folgende Schritte umfasst:
a. formschlüssiges Einsetzen eines Stiftes in die Bohrung; und
b. formschlüssiges Anordnen einer Bohrhülse über dem Stift.

12. Verfahren nach einem der vorhergehenden Ansprüche, wobei über der Bohrhülse eine weitere Bohrhülse formschlüssig angeordnet wird.

13. Verfahren nach einem der vorhergehenden Ansprüche, wobei als aushärtbare Formgießmasse ein Polymer verwendet wird.

14. Verfahren nach einem der vorhergehenden Ansprüche, wobei auf Basis des mit den Bohrungen versehenen Patientenkiefermodells ein Zahnersatz angefertigt wird.

15. Verfahren nach einem der vorhergehenden Ansprüche, wobei zum Bohren von wenigstens einer Bohrung in das Kiefermodell eine Bohreinrichtung (100) verwendet wird, welche aufweist:
a. eine Trageinheit (102), welche einen um einen ersten Aufhängungspunkt frei schwenkbaren Kiefermodellträger (103) aufweist; und
b. eine Bohreinheit (101), welche einen um einen zweiten Aufhängungspunkt frei schwenkbaren Bohrer (114) zum Bohren in ein auf dem Kiefermodellträger (103) angeordnetes Kiefermodell aufweist.

16. Verfahren nach Anspruch 15, wobei an dem Bohrer (114) ein mit der Mehrzahl der zweiten Reflexionskörper versehenes Trägerelement fixiert wird.

17. Verfahren nach Anspruch 16, wobei das Trägerelement eine im wesentlichen T-förmige Geometrie aufweist.

18. Verfahren nach Anspruch 17, wobei die zweiten Reflexionskörper an den Endpunkten des im wesentlichen T-förmigen Trägerelements angeordnet sind.

19. Bohrschablone für die Einbringung von Zahnimplantatbohrungen in einen Patientenkiefer, welche gemäß einem Verfahren nach einem der vorhergehenden Ansprüche hergestellt ist.

20. Zahnersatz, welcher an einem mittels eines Verfahrens nach einem der Ansprüche 1 bis 18 mit Bohrungen versehenen Patientenkiefermodell hergestellt worden ist.

21. Bohreinrichtung zum Bohren von wenigstens einer Bohrung in ein Kiefermodell, mit:
a. einer Trageinheit (102), welche einen um einen ersten Aufhängungspunkt frei schwenkbaren Kiefermodellträger (103) aufweist; und
b. einer Bohreinheit (101), welche einen um einen zweiten Aufhängungspunkt frei schwenkbaren Bohrer (114) zum Bohren in ein auf dem Kiefermodellträger (103) angeordnetes Kiefermodell aufweist.
